# NEUE EUROPÄISCHE PATENTSCHRIFT

(11) **EP 1 658 234 B2**
(45) Veröffentlichungstag und Bekanntmachung des Hinweises auf die Entscheidung über den Einspruch: **13.05.2015**
(45) Hinweis auf die Patenterteilung: 06.05.2009
(21) Anmeldenummer: 04764197.2
(22) Anmeldetag: 17.08.2004
(51) Int. Cl.: A61K 8/22, A61Q 5/08, A61Q 5/10, C01B 15/037, C11D 3/39

(54) **STABILISIERUNG VON WASSERSTOFFPEROXID WÄHREND DER AUFLÖSUNG ALKALISIEREND WIRKENDER FESTSTOFFE IN WASSERSTOFFPEROXIDHALTIGEN SYSTEMEN**
STABILISATION OF HYDROGEN PEROXIDE DURING DISSOLVING ALKALISING AGENT IN HYDROGEN PEROXIDE-CONTAINING SYSTEMS
STABILISATION DE PEROXYDE D'HYDROGENE LORS DE LA DISSOLUTION DE SUBSTANCES ALCALINISANTES DANS DES SYSTEMES CONTENANT DU PEROXYDE D'HYDROGENE

(30) Priorität: 26.08.2003 DE 10339164
(43) Veröffentlichungstag der Anmeldung: 24.05.2006
(73) Patentinhaber: Henkel AG & Co. KGaA, 40589 Düsseldorf (DE)
(72) Erfinder: HÖFFKES, Horst, 40595 Düsseldorf (DE); SEILER, Martina, 47228 Duisburg (DE); PAULI, Kristin, 42119 Wuppertal (DE)
(86) Internationale Anmeldenummer: PCT/EP2004/009207
(87) Internationale Veröffentlichungsnummer: WO 2005/021427

(56) Entgegenhaltungen:
- WO-A-94/03553
- WO-A-95/23210
- WO-A1-00/20550
- DE-A- 3 814 685
- US-A- 5 674 476
- US-A- 5 998 663
- US-B1- 6 540 791
- "Zeolithe" RÖMPP Online, Version 3.6

## Beschreibung

Gegenstand der vorliegenden Erfindung ist eine feste, alkalisierend wirkende Zusammensetzung, welche zu mindestens 75 Gew.% bezogen auf das Gewicht der Zusammensetzung ein Gemisch aus mindestens einem Alkalisierungsmittel und mindestens einem Chelatbildner enthält, sowie die Verwendung dieser Zusammensetzung zur Verminderung der Zersetzung von Wasserstoffperoxid während des Auflösevorgangs von festen oder pastösen Komponenten, enthaltend als Alkalisierungsmittel die erfindungsgemäße feste, alkalisierend wirkende Zusammensetzung, wobei das Lösemittel und/oder die zu lösende Komponente Wasserstoffperoxid enthält.

In vielfältigen Prozessen finden wasserstoffperoxidhaltige Mittel Anwendung, wie beispielsweise bei der oxidativen Behandlung von Fasern jeglicher Art, der Aufhellung und Reinigung von Textilien oder Oberflächen, der Dauerverformung oder der dauerhaften Farbveränderung bei einer oxidativen Färbung bzw. einer Bleiche von Fasern z.B. von keratinhaltigen Fasern.

Zum Bleichen, wie beispielsweise beim Aufhellen von Textilien oder Blondieren menschlicher Haare - bei letzterem insbesondere für die Strähnchenapplikation - werden üblicherweise feste oder pastenförmige Zubereitungen mit festen Oxidationsmitteln, den sogenannten "Bleich-Boostern", unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Für dauerhafte, intensive Färbungen von Fasern, insbesondere keratinhaltigen Fasern, mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß der vor der Anwendung zugefügten Oxidationsmittel untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Temporäre Färbungen werden auf einer Faser erzielt, wenn sogenannte direktziehende Farbstoffe in Färbemitteln zum Einsatz kommen. Direktziehende Farbstoffe sind an sich farbig und benötigen kein Oxidationsmittel zur Farbbildung. Die direktziehenden Farbstoffe können jedoch auch zusammen mit oxidativen Färbemitteln zur gezielten Beeinflussung der Farbnuance verwendet werden.

Es sind weiterhin Färbeverfahren bekannt, in denen als einzige farbgebende Komponente direktziehende Farbstoffe in Kombination mit wasserstoffperoxidhaltingen Mitteln, wie z.B. den vorstehenden Mitteln zur Bleiche, eine Farbveränderung bewirken. Dabei ist das wasserstoffperoxidhaltige Mittel für eine Aufhellung der Faser verantwortlich, wodurch die erhaltene Färbung der Faser brillanter erscheint als bei einer Ausfärbung mit den direktziehenden Farbstoffen allein.

Oftmals werden die wasserstoffperoxidhaltigen Mittel kurz vor der Anwendung aus getrennt gelagerten Zusammensetzungen unter Erhalt der eigentlichen Anwendungsmischung vermengt. Dieses Vorgehen ist besonders dann notwendig, wenn die Anwendungsmischung neben dem Wasserstoffperoxid solche Komponenten enthält, die mit dem Wasserstoffperoxid eine chemische Reaktion eingehen. Die eigentliche Anwendungsmischung ist aus diesem Grund nicht lagerstabil.

Direktziehende Farbstoffe sind beispielsweise des öfteren nicht stabil gegenüber Wasserstoffperoxid. Sollen sie zusammen mit Wasserstoffperoxid zum Einsatz kommen, werden sie erst kurz vor der Anwendung zu einer wasserstoffperoxidhaltigen Zusammensetzung gemischt. Da die Oxidationsfarbstoffvorprodukte sowie die direktziehenden Farbstoffe überwiegend Feststoffe sind, können sie kurz vor der Anwendung in fester Form als Pulver, Granulat oder Tablette zu der wasserstoffperoxidhaltigen Zusammensetzung gegeben werden.

Der Wirkungsgrad, wie z.B. die Aufhelleistung von o.g. Bleichmitteln, bzw. die Färbeleistung von o.g. Färbemitteln, ist bei einem basischen pH-Wert, insbesondere von einem pH zwischen 8 und 12, am größten. Allerdings ist bei einem solchen basischen pH-Wert eine Wasserstoffperoxidzubereitung nicht lagerstabil. Lagerstabile Wasserstoffperoxidzubereitungen besitzen einen neutralen, meist einen sauren pH-Wert von pH 2 bis 5. Um zu einer basischen Anwendungsmischung zu gelangen, enthält die beizumischende Komponente Alkalisierungsmittel.

Ferner ist es bekannt, daß neben einem basischen pH-Wert die Gegenwart von Zersetzungsbeschleunigern, wie Metallkationen, Zeolithen oder Bleich-Boostern die Zersetzung von Wasserstoffperoxid verstärkt.

Im Allgemeinen findet bei der Auflösung von festen, alkalisierend wirkenden Zusammensetzungen in einer wäßrigen, als Lösemittel fungierenden flüssigen Zusammensetzung während des Auflösevorgangs eine Zersetzung des in der flüssigen und/oder der festen Zusammensetzung enthaltenen Wasserstoffperoxids statt. Die H₂O₂-Zersetzung ist besonders ausgeprägt, wenn die wäßrige, als Lösemittel fungierende Zusammensetzung viskos ist. Eine solche Zersetzung wird von einer Sauerstoffentwicklung begleitet und tritt schlimmstenfalls als exotherme chemische Reaktion in Erscheinung. Die Gasentwicklung bewirkt in einem H₂O₂- sowie schaumbildnerhaltigen Mittel eine Schaumbildung. Neben der Zersetzung des Wasserstoffperoxids ist auch diese Schaumbildung unerwünscht, da der Schaum die Diffusion der Wirkstoffe in die Faser beeinträchtigt. Findet der Auflösevorgang wie üblich in einem verschlossenen Gefäß durch schütteln statt, resultiert aus der Gasentwicklung ein Überdruck in diesem Gefäß. Bei Öffnung des Gefäßes nach dem Auflösevorgang spritzt schlimmstenfalls durch den zeitgleich stattfindenden Druckausgleich die Anwendungmischung unkontrolliert aus dem Gefäß, was eine Gefahr für den Anwender bedeuten kann.

Die Zersetzung des Wasserstoffperoxids senkt folglich den Wirkungsgrad der Anwendungsmischung und erhöht das Gefahrenpotenzial beim Umgang mit wasserstoffperoxidhaltigen Mitteln. Bekanntermaßen werden daher zur Stabilisierung von Wasserstoffperoxid Chelatbildner in wasserstoffperoxidhaltigen Zusammensetzungen eingearbeitet. Diese Stabilisatoren können in den zuvor beschriebenen Mehrkomponentenmitteln sowohl Bestandteil des flüssigen Lösemittels, als auch eines zu lösenden Feststoffs sein. Allerdings reicht bloßes Beimengen der Chelatbildner nicht aus, um bei dem Lösevorgang eine Wasserstoffperoxidzersetzung ausreichend zu vermindern.

Die Druckschrift WO-A1-94/03553 betrifft feste oder flüssige Bleichmittel, die Wasserstoffperoxid, eine Wasserstoffperoxid freisetzende Substanz sowie 1,2-Ethylendiamin-N,N'-disuccinat bzw. dessen freie Säure als H₂O₂-Stabilisator enthalten.

In der Druckschrift WO-A1-95/23210 werden feste Partikel beschrieben, in deren Kern sich eine Wasserstoffperoxid freisetzende Substanz, wie beispielsweise Natriumpercarbonat oder eine Peroxysäure, befindet und welche mit einer Hydroxycarbonsäure als Chelatbildner beschichtet ist. Die Lagerstabilität der festen Partikel wird durch das Coating verbessert.

Die Aufgabe der vorliegenden Erfindung ist es, bei der Herstellung oxidativ wirkender, H₂O₂-haltiger Mittel aus mindestens einer flüssigen und mindestens einer alkalisierend wirkenden festen oder pastenförmigen Komponente, die Zersetzung von Wasserstoffperoxid während des Auflösevorgangs der alkalisierend wirkenden, festen oder pastenförmigen Komponente in der flüssigen Komponente zu verhindern bzw. gegenüber den Mitteln des Standes der Technik stark zu reduzieren. Das Wasserstoffperoxid ist hierbei in mindestens einer der vorgenannten Komponenten als zwingender Bestandteil enthalten.

Es wurde nun überraschenderweise gefunden, daß sich die Wasserstoffperoxidzersetzung während des Auflösevorgangs alkalisierend wirkender Zusammensetzungen in Gegenwart von H₂O₂ stark reduzieren läßt, wenn die zu lösende Zusammensetzung als alkalisierendes Agens eine alkalisierend wirkende, feste Zusammensetzung enthält, die als Agglomerat neben mindestens einem Alkalisierungsmittel mindestens einen Chelatbildner enthält. Die Chelatbildner befinden sich mit dem Alkalisierungsmittel in demselben Partikel des Agglomerats. Solche alkalisierend wirkenden Zusammensetzungen sind neu.

Eine Zusammensetzung ist im Sinne der Erfindung fest, wenn sie bei 20°C bei einem Druck von 101325 Pa als Feststoff vorliegt.

Ein Agglomerat im Sinne der Erfindung ist eine Zusammenballung von mehreren verschiedenen Stoffen unter Bildung fester Partikel.

Eine alkalisierend wirkende Zusammensetzung vermag es im Sinne der Erfindung in einer Konzentration von mindestens 10⁻² mol/l den pH-Wert eines wäßrigen oder wäßrig-alkoholischen Systems auf einen pH von größer 7 zu erhöhen.

Ein wäßriges System, bzw. ein wäßriger Träger, enthält im Sinne der Erfindung mindestens 10 Gew.% Wasser. Als wasserfrei werden Systeme bezeichnet, die weniger als 10 Gew.% Wasser enthalten. Unter wäßrig-alkoholischen Systemen bzw. wäßrig-alkoholischen Trägern, sind im Sinne der vorliegenden Erfindung wäßrige Systeme enthaltend 3 bis 70 Gew.-% eines gegebenenfalls substituierten C₁-C₄-Alkohols mit mindestens einer Hydroxygruppe, wie beispielsweise Methoxybutanol, Benzylalkohol, Ethyldiglykol oder 1,2-Propylenglykol, Glycerin sowie insbesondere Ethanol bzw. Isopropanol, zu verstehen.

Unter keratinhaltigen Fasern werden im Rahmen dieser Anmeldung Pelze, Wolle, Federn und insbesondere menschliche Haare verstanden.

Der Begriff des Chelatbildners ist dem Fachmann bekannt. Es wird in diesem Zusammenhang explizit auf das RÖMPP Chemie Lexikon, 9. erweiterte und neu bearbeitete Auflage, Georg Thieme Verlag, Stuttgart, 1995, Band 1 (A-Cl), Seite 634 verwiesen.

Ein erster Gegenstand der Erfindung ist eine alkalisierend wirkende, feste Zusammensetzung, die
(i) in einer Menge von mindestens 75 Gew._% bezogen auf das Gewicht der Zusammensetzung ein Gemisch aus (a) mindestens einem partikulären Alkalisierungsmittel sowie (b) mindestens einem Chelatbildner und
(ii) gegebenenfalls weitere Zusatzstoffe enthält,
wobei die Zusammensetzung aus Agglomeraten, gebildet aus (a), (b) und den optional enthaltenen Zusatzstoffen, besteht, dadurch gekennzeichnet, dass der Chelatbildner ausgewählt wird aus Ethylendiamintetraessigsäure (EDTA), Diethylentriaminpentaessigsäure, 1-Hydroxyethan-1,1-diphosphonsäure (Etidronsäure), Dipicolinsäure, Acetanilid und Natriumstannat, sowie den physiologisch verträglichen Salzen der vorgenannten Säuren.

Die Herstellung der Agglomerate der erfindungsgemäßen alkalisierend wirkenden, festen Zusammensetzung kann auf unterschiedliche Weise erfolgen. Die Alkalisierungsmittel und die Chelatbildner können bei dieser Herstellung sowohl als Feststoffe als auch als Flüssigkeiten zum Einsatz kommen. Bei der Verwendung zweier Feststoffe zur Herstellung der Agglomerate, werden diese durch bekannte Verfahren der Preßagglomeration zu Agglomeraten zusammengeballt. Dabei kann es bevorzugt sein, zusätzlich sogenannte Bindemittel, wie beispielsweise Bentonit, Melasse, Öle oder Wachse, einzusetzen. Bevorzugt werden zur Bildung der Agglomerate der Chelatbildner und der Alkalisierungmittel die beiden festen Komponenten zunächst zusammen in einem Lösemittel, beispielsweise Wasser, gelöst, um anschließend das Lösemittel zu entfernen. Die Lösemittel werden dabei in bekannten Trocknungsverfahren, wie z.B. einem Walzentrocknungsverfahren oder der Rieseltrocknung, unter Erhalt des erfindungsgemäßen alkalisierend wirkenden, festen Zusammensetzung entfernt.

Wenn entweder das Alkalisierungsmittel oder der Chelatbildner als Feststoff und die andere Komponente als Flüssigkeit oder Lösung verwendet wird, so ist die Dosierung der Flüssigkeit derart zu wählen, daß nach der Behandlung des Feststoffs mit der flüssigen Komponente ein fließfähiges Pulver zurückbleibt. Bevorzugt wird die flüssige Komponente durch eine geeignete Vorrichtung auf die feste Komponente aufgesprüht.

Die wie oben beschrieben erhaltenen alkalisierend wirkenden, festen Zusammensetzungen können darüber hinaus in üblichen Verfahren kompaktiert, extrudiert oder granuliert werden.

Die Chelatbildner können innerhalb des Agglomerats unterschiedlich räumlich verteilt sein. So können sich die Chelatbildner an der Oberfläche von Agglomeraten des Alkalisierungsmittels als sogenannte Oberflächenbeschichtung bzw. Coating befinden. Die Partikel des Alkalisierungsmittels und des Chelatbildners können jedoch ebenso gleichverteilt in dem Agglomerat vorliegen.

Die Agglomerate haben bevorzugt einen mittleren Teilchendurchmesser von 10 bis 300 µm, besonders bevorzugt von 100 bis 200 µm.

Die optionalen Zusatzstoffe können neben den zuvor genannten Bindemitteln bevorzugt diejenigen Zusatzstoffe sein, die in den nachstehend definierten Ausführungsformen der erfindungsgemäßen alkalisierend wirkenden, festen Zusammensetzung enthalten sein können.

Erfindungsgemäß können die dem Fachmann bekannten, üblichen partikulären Alkali**sierungsmittel** wie Ammonium-, Alkalimetall- und Erdalkalimetallhydroxide, -carbonate, -hydrogencarbonate, -hydroxycarbonate, -carbamide, -silikate, insbesondere - metasilikate, sowie Alkaliphosphate verwendet werden. In einer bevorzugten Ausführungsform enthält die erfindungsgemäße alkalisierend wirkende, feste Zusammensetzung mindestens zwei unterschiedliche partikuläre Alkalisierungsmittel. Dabei können Mischungen beispielsweise aus einem Metasilikat und einem Hydroxycarbonat bevorzugt sein.

In einer besonders bevorzugten Ausführungsform enthält die erfindungsgemäße alkalisierend wirkende, feste Zusammensetzung mindestens ein Metasilikat des Ammoniums oder der Alkali- bzw. Erdalkalimetalle als Alkalisierungsmittel. Ganz besonders bevorzugte erfindungsgemäße Metasilikate sind Wassergläser, die aus einer wäßrigen Lösung eines Silikates der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Einsetzbar sind prinzipiell auch amorphe Natriumsilikate mit einem Modul Na₂O SiO2 von 1:2 bis 1:3,3, vorzugsweise von 1:2 bis 1:2,8 und insbesondere von 1:2 bis 1:2,6, welche löseverzögert sind. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Eigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Fa. Henkel unter den Bezeichnungen Ferrosil^{®} 119, Natronwasserglas 40/42, Portil^{®} A, Portil^{®} AW, Portil^{®} N und Portil^{®} W und von der Firma PQ Nederlands unter der Bezeichnung Britesil^{®} C20 vertrieben.

Die partikulären Alkalisierungsmittel sind bevorzugt in einer Menge von 80 bis 99.8 Gew.%, besonders bevorzugt in einer Menge von 90 bis 98 Gew.%, jeweils bezogen auf das Gewicht der gesamten Zusammensetzung, in den erfindungsgemäßen alkalisierend wirkenden festen Zusammensetzungen enthalten.

Dem Fachmann sind eine Vielzahl **Chelatbildner** aus der einschlägigen Literatur bekannt. Besonders geeignet sind Polycarbonsäuren und deren wasserlösliche Natrium-, Kalium-, Magnesium- und Ammoniumsalze, verschiedene Fruchtsäuren, Derivate von Aminosäuren, Aminopolycarbonsäuren, Metaphosphor-, Polyphosphor- und Polyphosphonsäuren und deren Salze, Alkalimetallstannate, wie z.B. Natriumstannat, Hydroxycarbonsäuren und deren Salze, wie z. B. Citronensäure, Weinsäure, Äpfelsäure und Gluconsäure, Glucuronsäure, Galactarsäure, sowie Benzamide und Anilide, wie z.B. Acetanilid, und die α-Hydroxycarbonsäuren gemäß WO-A1-95/23210, auf die ausdrücklich Bezug genommen wird.

Beispiele für erfindungsgemäße Polycarbonsäuren sind Bernsteinsäure, 1,2,3-Propantricarbonsäure, Dipicolinsäure, Cyclodextrine, β-Alanindiessigsäure und deren Salze, Dihydroxyethylglycinate, Dicarboxymethylalaninate, Tetrahydroxyethyl- und Tetrahydroxypropylethylendiamin sind ebenso erfindungsgemäße Chelatbildner.

Geeignet als Chelatbildner sind des weiteren auch die wasserlöslichen Salze von Aminopolycarbonsäuren sowie deren Natrium-, Kalium-, Ammonium-, Magnesium-, Calcium und Triethanolaminsalze. In der erfindungsgemäßen Zusammensetzung des ersten Gegenstandes bevorzugt eingearbeitete Aminocarbonsäuren sind Ethylendiamintetraessigsäure (EDTA) und deren Salze, wie z. B. Calcium-Dinatrium-EDTA, Diammonium-EDTA, Dinatrium- und Dikalium-EDTA, Triethanolamin-EDTA, Trinatrium- und Trikalium-EDTA, Tetranatrium- und Tetrakalium-EDTA, sowie Hydroxyethylethylendiamintriessigsäure, Cyclohexandiamintetraessigsäure, Diethylentriaminpentaessigsäure, Lauroylethylendiamintriessigsäure, Ethylendiamindibernsteinsäure und Dipicolinsäure und die entsprechenden Salze. Erfindungsgemäß besonders bevorzugt als Aminopolycarbonsäure ist Tetranatrium-EDTA und Trinatrium-EDTA, welche beispielsweise unter der Bezeichnung Trilon® B und Trilon® A im Handel sind. Weitere bevorzugte erfindungsgemäße Aminopolycarbonsäuren sind 1,2-Ethylendiamin-N,N'-diglutarsäure (EDDG) sowie Iminodisuccinate wie beispielsweise 1,2-Ethylendiamin-N,N'-disuccinat (EDDS), und 2-Hydroxypropylendiamin-N,N'-disuccinat (HPDDS).

Bevorzugt verwendete Polyphosphorsäuren bzw. Salze davon sind solche der Formel M⁺ₙ₊₂₋ₓ [HₓPₙO₃ₙ₊₁]^{(n+2-x)-}, worin M bevorzugt für Wasserstoff, Natrium oder Kalium steht und n eine natürliche Zahl ungleich Null und 1 ist und x ist eine natürliche Zahl von 0 bis 3 Beispiele für solche erfindungsgemäßen Polyphosphorsäuren bzw. deren Salze sind Tetranatriumdiphosphat, Pentanatriumtriphosphat und Dinatriumdihydrogendiphosphat.

Erfindungsgemäß sind darüber hinaus ebenso die zyklischen meta-Phosphorsäuren bzw. deren Salze mit der Formel M⁺ₙ [PₙO₃ₙ₊₁]ⁿ⁻, wobei n eine natürliche Zahl ungleich Null ist. Beispiel einer bevorzugten Metaphosphorsäure ist das Trinatriummetaphosphat oder das Natriummetaphosphat das beispielsweise unter dem Handelsnamen Calgon^{®} vertieben wird.

Beispiele für erfindungsgemäße Polyphosphonsäuren sind 1-Hydroxyethan-1,1-diphosphonsäure (Etidronsäure), N,N,N-Tri(phosphonomethyl)amin, 1,2-Ethylendiamintetramethylenphosphonsäure (EDTMP), Diethylentriamin-pentamethylenphosphonsäure (DTPMP), N,N,N-Tri(1-phosphono-ethyl)-amin, N,N,N-Tri(1-phosphono-propyl)amin und N,N,N-Tri(2-phosphono-prop-2-yl)-amin. Etidronsäure ist eine bevorzugte Polyphosphonsäure.

Besonders bevorzugt werden die Chelatbildner ausgewählt aus Ethylendiamintetraessigsäure (EDTA), Diethylentriaminpentaessigsäure, 1-Hydroxyethan-1,1-diphosphonsäure (Etidronsäure), Dipicolinsäure, Acetanilid und Natriumstannat, sowie den physiologisch verträglichen Salzen der vorgenannten Säuren.

Die Chelatbildner sind bevorzugt in einer Menge von 0.1 bis 20 Gew.%, besonders bevorzugt in einer Menge von 2 bis 10 Gew.%, jeweils bezogen auf das Gewicht der gesamten Zusammensetzung, in diesen erfindungsgemäßen alkalisierend wirkenden Zusammensetzungen enthalten.

Das Gemisch aus Alkalisierungsmittel und Chelatbildner ist bevorzugt zu mindestens 90 Gew.%, besonders bevorzugt zu mindestens 97 Gew.%, bezogen auf das Gewicht der gesamten erfindungsgemäßen alkalisierend wirkenden, festen Zusammensetzung, enthalten.

Die erfindungsgemäßen alkalisierend wirkenden festen Zusammensetzungen können weitere kosmetische Zusatzstoffe enthalten. Diese Zusatzstoffe sind solche, die in den Zusammensetungen (A), (B) und (C) des zweiten Gegenstandes der Erfindung enthalten sein können. Bevorzugte kosmetische Zusatzstoffe sind Tenside, konditionierende Wirkstoffe, Farbstoffe sowie Farbstoffvorprodukte. Die kosmetischen Zusatzstoffe sind in Summe in der alkalisierend wirkenden, festen Zusammensetzung bevorzugt zu 0 bis 25 Gew.%, besonders bevorzugt von 0 bis 10 Gew.%, ganz besonders bevorzugt von 0 bis 3 Gew.%, jeweils bezogen auf das Gewicht der Zusammensetzung, enthalten.

Ein zweiter Gegenstand der vorliegenden Erfindung ist ein wasserstoffperoxidhaltiges Mittel, das durch Mischen von mindestens zwei getrenntkonfektionierten Zusammensetzungen (A) und (B) erhalten wird, dadurch gekennzeichnet, daß
(i) die Zusammensetzung (A) wasserfrei ist und mindestens eine alkalisierend wirkende, feste Zusammensetzung des ersten Erfindungsgegenstandes enthält,
(ii) die Zusammensetzung (B) wäßrig oder wäßrig-alkoholisch ist und
(iii) mindestens eine der Zusammensetzungen (A) und (B) Wasserstoffperoxid enthält.

Das in den erfindungsgemäßen Zusammensetzungen (A) und/oder (B) enthaltene Wasserstoffperoxid wird erfindungsgemäß als Lösung oder in Form einer festen Anlagerungsverbindung von Wasserstoffperoxid an anorganische oder organische Verbindungen, wie beispielsweise Natriumperborat, Natriumpercarbonat, Natriumpercarbamid, Polyvinylpyrrolidon-n H₂O₂ (n ist eine positive ganze Zahl größer 0), Harnstoffperhydrat und Melaminperhydrat, der Zusammensetzung (A) bzw. (B) zugegeben.

Wenn Wasserstoffperoxid ein Bestandteil der wasserfreien Zusammensetzung (A) ist, liegt es als dispergierter, partikulärer Feststoff in Form einer festen Anlagerungsverbindung von Wasserstoffperoxid an anorganische oder organische Verbindungen vor. Während des Vermischens mit der Zusammensetzung (B) wird durch den Kontakt mit Wasser gelöstes Wasserstoffperoxid gebildet.
In einer bevorzugten Ausführungsform enthält nur eine der Zusammensetzungen (A) oder (B) Wasserstoffperoxid.

In einer bevorzugten Ausführungsform ist als einziges Alkalisierungsmittel des wasserstoffperoxidhaltigen Mittels, die erfindungsgemäße alkalisierend wirkende, feste Zusammensetzung enthalten.

Das erfindungsgemäße Mittel besitzt bevorzugt einen pH-Wert von größer 7.

**Die Zusammensetzung (A)** ist bevorzugt fest oder pastös. Die pastöse Form kann beispielsweise durch Mischen der festen Komponenten mit Ölen und/oder flüssigen und wasserfreien nichtionogenen Tensiden erhalten werden. Bevorzugte Öle sind hierbei unter anderem natürliche und synthetische Öle, geradkettige und verzweigte Kohlenwasserstoffe und flüssige Wachse, sowie Silikonöle (gemäß EP-A1-560 088, auf die vollinhaltlich Bezug genommen wird, z.B. Paraffinöl), Dialkylether (wie sie beispielsweise in der Druckschrift DE-A1-196 00 216 offenbart sind, auf die vollinhaltlich Bezug genommen wird, z.B Di-n-octylether und Di-n-dodecylether), Carbonsäure- und Kohlensäureester.

Ferner enthält die Zusammensetzung (A) die erfindungsgemäße alkalisierend wirkende, feste Zusammensetzung bevorzugt in einer Menge von 1 bis 40 Gew.%, besonders bevorzugt in einer Menge von 2 bis 30 Gew.% jeweils bezogen auf das Gewicht der gesamten Zusammensetzung (A).

Bevorzugt enthält die Zusammensetzung (A) mindestens einen Bleich-Booster. Feste Peroxoverbindungen, die keine Anlagerungsprodukte von Wasserstoffperoxid an andere Komponenten darstellen, sind insbesondere bei der Bleiche keratinhaltiger Fasern vorteilhaft. Die Auswahl der in der erfindungsgemäßen Zusammensetzung (A) enthaltenen Peroxoverbindungen unterliegt prinzipiell keinen Beschränkungen; übliche, dem Fachmann bekannte Peroxoverbindungen sind beispielsweise (i) Peroxodisulfate, Persulfate und Peroxodiphosphate, wie beispielsweise Ammoniumperoxodisulfat, Kaliumperoxodisulfat, Natriumperoxodisulfat, Ammoniumpersulfat, Kaliumpersulfat, Natriumpersulfat, Kaliumperoxodiphosphat, und weiterhin (ii) Peroxide der Alkali- und Erdalkalimetalle, wie Magensium- und Bariumperoxid, sowie (iii) Peroxocarbonsäuren oder deren physiologisch verträglichen Salze, wie z.B. Magnesiumperphthalat. Unter diesen Peroxoverbindungen, die auch in Kombination eingesetzt werden können, sind erfindungsgemäß die anorganischen Verbindungen bevorzugt. Besonders bevorzugt sind die Peroxodisulfate, insbesondere Ammoniumperoxodisulfat.

Als Bleich-Booster können weiterhin, insbesondere bei der Bleiche bzw. beim Waschen von Textilfasern jeglicher Art, Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Weitere bevorzugte Bleichaktivatoren sind (iv) kationische Nitrile, insbesondere der nachstehenden Formel, 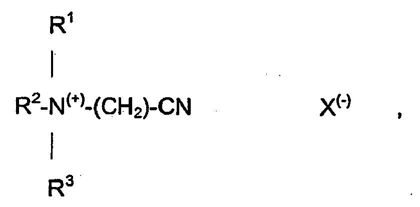 in der R¹ für -H, -CH₃, einen C₂₋₂₄-Alkyl- oder -Alkenylrest, einen substituierten C₂₋₂₄-Alkyl- oder -Alkenylrest mit mindestens einem Substituenten aus der Gruppe -Cl, -Br, - OH, -NH₂, -CN, einen Alkyl- oder Alkenylarylrest mit einer C₁₋₂₄-Alkylgruppe, oder für einen substituierten Alkyl- oder Alkenylarylrest mit einer C₁₋₂₄-Alkylgruppe und mindestens einem weiteren Substituenten am aromatischen Ring steht, R² und R³ unabhängig voneinander ausgewählt sind aus -CH₂-CN, -CH₃, -CH₂-CH₃, -CH₂-CH₂-CH₃, -CH(CH₃)-CH₃, -CH₂-OH, -CH₂-CH₂-OH, -CH(OH)-CH₃, -CH₂-CH₂-CH₂-OH, -CH₂-CH(OH)-CH₃, - CH(OH)-CH₂-CH₃, -(CH₂CH₂-O)ₙH mit n = 1, 2, 3, 4, 5 oder 6 und X ein Anion ist.

Unter diese allgemeine Formel fällt eine Vielzahl von kationischen Nitrilen, die im Rahmen der vorliegenden Erfindung einsetzbar sind. Mit besonderem Vorteil enthalten die erfindungsgemäßen Zusammensetzung dabei kationische Nitrile, in denen R¹ für Methyl, Ethyl, Propyl, Isopropyl oder einen n-Butyl, n-Hexyl, n-Octyl, n-Decyl, n-Dodecyl, n-Tetradecyl, n-Hexadecyl oder n-Octadecylrest steht. R² und R³ sind vorzugsweise ausgewählt aus Methyl, Ethyl, Propyl, Isopropyl und Hydroxyethyl, wobei einer oder beide Reste vorteilhaft auch noch ein Cyanomethylenrest sein kann.

Die Bleich-Booster sind in der erfindungsgemäßen Zusammensetzung (A) bevorzugt in Mengen von 5 bis 60 Gew.-%, insbesondere in Mengen von 8 bis 30 Gew.-%, jeweils bezogen auf das Gewicht der gesamten Zusammensetzung (A), enthalten.

**Die Zusammensetzung (B)** ist ein wäßriges oder wäßrig-alkoholisches System. Sie kann in Form einer Emulsion, z.B. einer W/O- oder O/W-Emulsion, vorliegen. Die Viskosität der Zusammensetzung (B) liegt bevorzugt in einem Bereich von 1 bis 100000 mPa·s, bevorzugt von 1000 bis 70000 mPa·s, besonders bevorzugt von 6000 bis 50000 mPa·s und ganz besonders bevorzugt von 10000 bis 30000 mPa·s. Die Viskositäten werden mit einem Brookfield RVT-Viskosimeter bei einer Temperatur von 20 °C bei 4 rpm mit Spindel Nr. 4 gemessen. Die Wahl der Spindel zur Messung der oben genannten Viskositäten erfolgt jedoch bevorzugt je nach Viskositätsbereich (gemessen bei den oben genannten Versuchsbedingungen) gemäß Tabelle 1.

**Tabelle 1**

| **Spindel Nummer** | **Viskositätsbereich [mPa·s]** |
|---|---|
| 1 | bis 2500 |
| 2 | > 2500 bis 10000 |
| 3 | > 10000 bis 25000 |
| 4 | > 25000 bis 50000 |
| 5 | > 50000 bis 100000 |

In einer speziellen Ausführungsform hat die Zusammensetzung (B) eine Viskosität von 1 bis 50000 mPa·s, besonders bevorzugt von 500 bis 25000 mPa·s, ganz besonders bevorzugt von 500 bis 15000 mPa·s. Die Messung der Viskositäten dieser speziellen Ausführungsform wird mit einem Brookfield RVT-Viskosimeter bei 20°C mit der Spindel 4 und 20 rpm durchgeführt.

Die Zusammensetzung (B) besitzt bevorzugt einen pH-Wert in einem Bereich von pH 2 bis 7, besonders bevorzugt in einem Bereich von pH 3 bis 6.

Die Zusammensetzung (B) kann zusätzlich mindestens einen Chelatbildner enthalten. Als bevorzugte Chelatbildner gelten die zuvor genannten Chelatbildner.

Die Zusammensetzungen (A) und (B) können zusätzlich mindestens ein Tensid enthalten, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.
   Beispiele für die in den erfindungsgemäßen Zusammensetzungen verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.
   Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).
   Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.
   Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".
   Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.
   Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren Erdalkalimetalloxide. -hydroxide oder -alkoholate als Katalysatoren verwendet werden. Die Verwendung von Produkten mit eingeengter Homologenverteilung kann bevorzugt sein.
   Weiterhin können die Zusammensetzungen (A) und/oder (B) bevorzugt noch einen **konditionierenden Wirkstoff,** ausgewählt aus der Gruppe, die von kationischen Tensiden, kationischen Polymeren, Alkylamidoaminen, Paraffinölen, pflanzlichen Ölen und synthetischen Ölen gebildet wird, enthalten.
   Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
   Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

In einer weiteren Ausführungsform enthalten die erfindungsgemäßen Zusammensetzungen (A) und/oder (B) zusätzlich strukturverbessernde Wirkstoffe. Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiele sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropan-diol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Zusammensetzungen geeignet sind die Extrakte aus Mandel, Aloe Vera, Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 bis 80 Gew.-% Aktivsubstanz und als Lösemittel das bei ihrer Gewinnung eingesetzte Extraktionsmittel oder Extraktionsmitteigemisch.

Weiterhin kann es bevorzugt sein, in den erfindungsgemäßen Zusammensetzungen Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1 bis 10 Gew.-%, insbesondere 3 bis 5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung: Polyquaternium-1) und Merquat^{®} 100 (INCI-Bezeichnung: Polyquaternium-6) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen und Hexosen, wie die entsprechenden On- und Uronsäuren (Zuckersäuren), Zuckeralkohole, Zuckeramine, wie beispielsweise N-Glucosamin, und Glykoside, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-y-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure ist besonders bevorzugt. Weiterhin verbessern spezielle Phosphonsäuren und ihre Salze die Struktur keratinhaltiger Fasern. Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1 - 10 C-Atomen unter Öffnung des Epoxidrings und Ausbildung einer vizinalen Dihydroxyethyl- oder Hydroxy-alkoxy-ethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5 bis 10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2 - 6 C-Atomen und 2-6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Zusammensetzungen als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol^{®} 760 erhältlich.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3aS,4S, 6aR)-2-Oxohexa-hydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Glycerinsäure, Vitamin B6, Niacinamid, Polyvinylpyrrolidon, Gluconsäure, Biotin und den lipidlöslichen Esteralkoholen oder Esterpolyolen.

Die erfindungsgemäßen Zusammensetzungen enthalten die strukturverbessernden Wirkstoffe bevorzugt in Mengen von 0,1 bis 5 Gew.-%, besonders bevorzugt in Mengen von 0,2 bis 2 Gew.-%.

In einer bevorzugten Ausführungsform der vorliegenden Erfindung enthalten die Zusammensetzungen weiterhin eine Magnesiumverbindung. Die erfindungsgemäßen Zusammensetzungen können durch Zugabe der Mg²⁺-Kationen hinsichtlich ihre strukturerhaltenden Eigenschaften weiter optimiert werden. Bevorzugte Magnesiumverbindungen sind anorganische und organische Mg²⁺-Salze, wie beispielsweise die Halogenide, die Carbonate und Hydrogencarbonate, das Acetat und das Citrat.
Wird ein Färbemittel formuliert, enthalten die erfindungsgemäßen Zusammensetzungen (A) oder (B)
- mindestens einen direktziehenden Farbstoff und/oder
- mindestens ein Farbstoffvorprodukt vom Entwicklertyp (Entwicklerkomponente) sowie gegebenenfalls mindestens ein Farbstoffvorprodukt vom Kupplertyp (Kupplerkomponente).

Als geeignete direktziehende Farbstoffe haben sich Nitrofarbstoffe erwiesen. Erfindungsgemäß sind unter Nitrofarbstoffen die färbenden Komponenten zu verstehen, die mindestens ein aromatisches Ringsystem aufweisen, das mindestens eine Nitrogruppe trägt.

Besonders bevorzugte Nitrofarbstoffe sind HC Yellow 2, HC Yellow 4, HC Yellow 5, HC Yellow 6, HC Yellow 12, HC Orange 1, HC Red 1, HC Red 3, HC Red 10, HC Red 11, HC Red 13, HC Red BN, HC Blue 2, HC Blue 12, HC Violet 1 sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Neben den Nitrofarbstoffen sind auch die Azofarbstoffe, Anthrachinone oder Naphthochinone erfindungsgemäß bevorzugte synthetische direktziehende Farbstoffe. Bevorzugte direktziehende Farbstoffe dieser Art sind beispielsweise Disperse Orange 3 Disperse Blue 3, Disperse Violet 1, Disperse Violet 4, Acid Violet 43, Disperse Black 9 und Acid Black 52 sowie 2-Hydroxy-1,4-naphthochinon.

Weiterhin kann es erfindungsgemäß bevorzugt sein, wenn der synthetische direktziehende Farbstoff eine kationische Gruppe trägt. Besonders bevorzugt sind
(i) kationische Triphenylmethanfarbstoffe,
(ii) aromatische Systeme, die mit einer quaternären Stickstoffgruppe substituiert sind, und
(iii) direktziehende Farbstoffe, die einen Heterocyclus enthalten, der mindestens ein quaternäres Stickstoffatom aufweist.

Beispiele für Farbstoffe der Klasse (i) sind insbesondere Basic Blue 7, Basic Blue 26, Basic Violet 2 und Basic Violet 14.
Beispiele für Farbstoffe der Klasse (ii) sind insbesondere Basic Yellow 57, Basic Red 76, Basic Blue 99, Basic Brown 16 und Basic Brown 17.
Beispiele für Farbstoffe der Klasse (iii) werden insbesondere in der EP-A2-998 908, auf die an dieser Stelle explizit Bezug genommen wird, in den Ansprüchen 6 bis 11 offenbart.
Bevorzugte kationische direktziehende Farbstoffe der Gruppe (iii) sind insbesondere die folgenden Verbindungen:

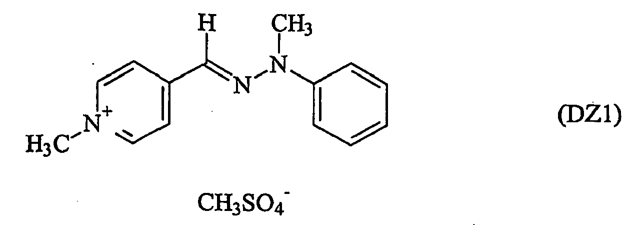

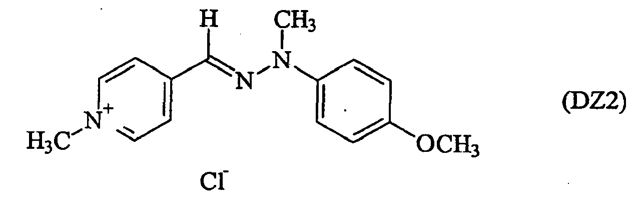

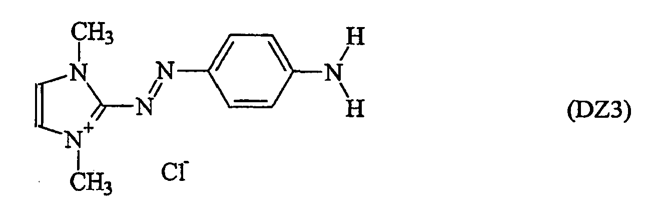

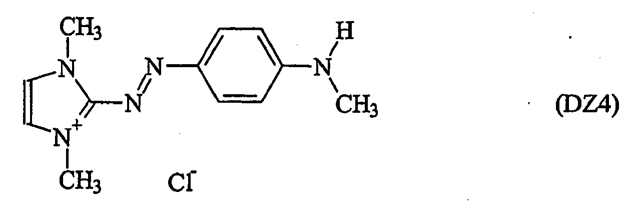

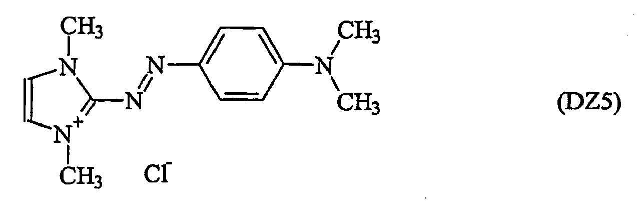

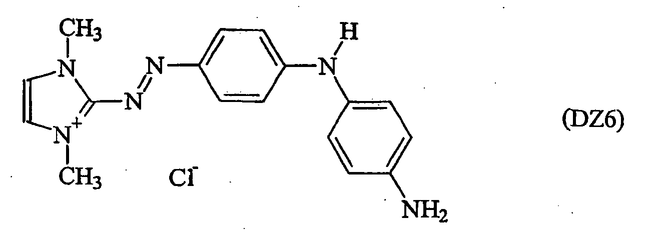

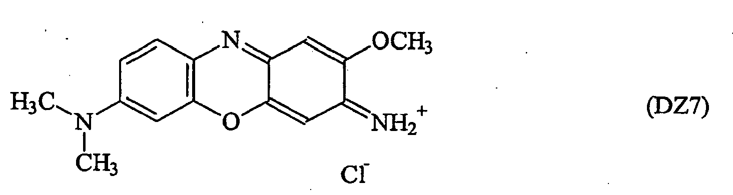

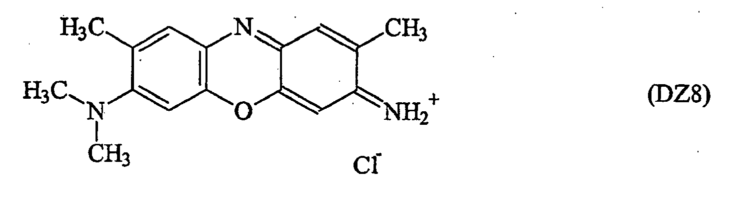

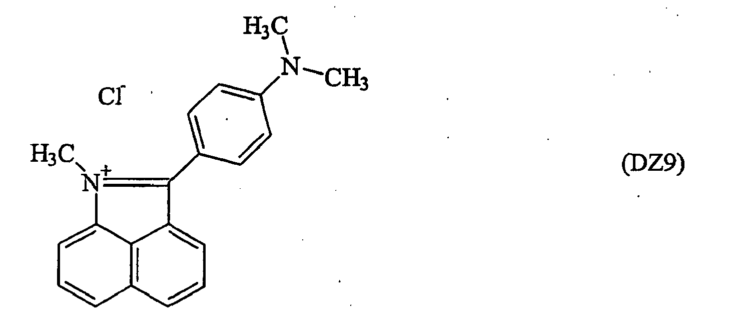

Die Verbindungen der Formeln (DZ1), (DZ3) und (DZ5) sind ganz besonders bevorzugte kationische direktziehende Farbstoffe der Gruppe (iii).

Weiterhin können die erfindungsgemäßen Zusammensetzungen (A) oder (B) auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Zusammensetzungen (A) oder (B) enthalten die direktziehenden Farbstoffe bevorzugt in einer Menge von 0,01 bis 20 Gew.-%, jeweils bezogen auf das gesamte Gewicht der jeweiligen, den direktziehenden Farbstoff enthaltenen Zusammensetzung.

Als **Entwicklerkomponenten** werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 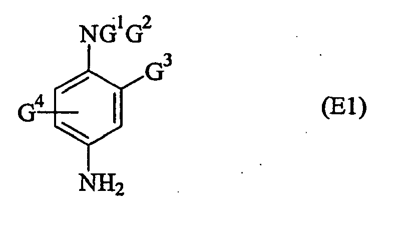 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C4-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, CI-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄- . Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Zusammensetzungen (A) oder (B) gemäß der Erfindung verwendet werden können, kann man insbesondere die Verbindungen nennen, die der folgenden Formel (E2) entsprechen, sowie ihre physiologisch verträglichen Salze: 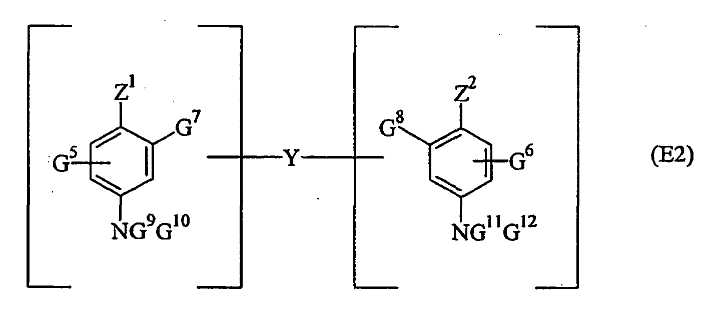 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
   mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N;N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 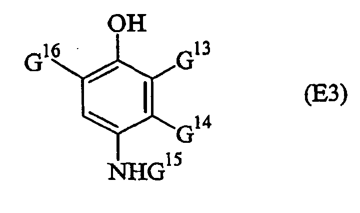 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁ bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hyd roxymethyl-1-isopropylpyrazol, 4,5-Diamino-3-methyl-1-isopropylpyrazol, 4-Amino-5-(□-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 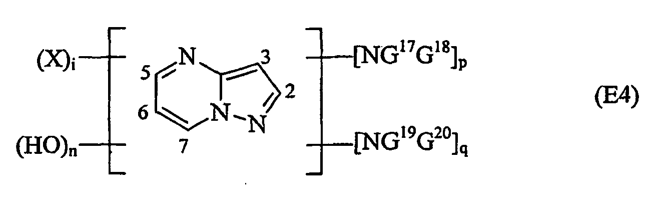 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

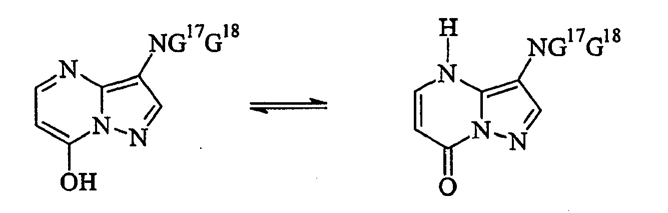

Unter den Pyrazolo[1,5-a]pyrimidinen der obenstehenden Formel (E4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Amino-pyrazolo[1,5-a]pyrimidin-7-ol;
- 3-Amino-pyrazolo[1,5-a]pyrimidin-5-ol;
- 2-(3-Amino-pyrazolo[1,5-a]pyrimidin-7-ylamino)-ethanol;
- 2-(7-Amino-pyrazolo[1,5-a]pyrimidin-3-ylamino)-ethanol;
- 2-[(3-Amino-pyrazolo[1,5-a]pyrimidin-7-yl)-(2-hydroxy-ethyl)-amino]-ethanol;
- 2-[(7-Amino-pyrazolo[1,5-a]pyrimidin-3-yl)-(2-hydroxy-ethyl)-amino]-ethanol;
- 5,6-Dimethyl-pyrazolo[1,5-a]pyrimidin-3,7-diamin;
- 2,6-Dimethyl-pyrazolo[1,5-a]pyrimidin-3,7-diamin;
- 3-Amino-7-dimethylamino-2,5-dimethyl-pyrazolo[1,5-a]pyrimidin;
   sowie ihre physiologisch verträglichen Salze und ihre tautomeren Formen, wenn ein tautomers Gleichgewicht vorhanden ist.
   Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.
   Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.
   Besonders gut als Vorstufen naturanaloger Haarfarbstoffe geeignet sind Derivate des 5,6-Dihydroxyindolins der Formel (IIa) als Entwicklerkomponente, 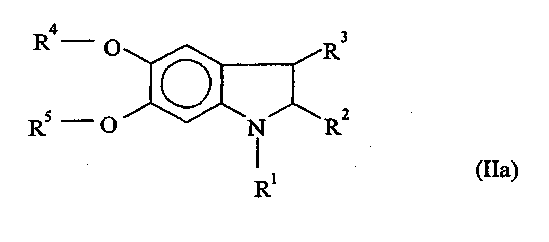 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 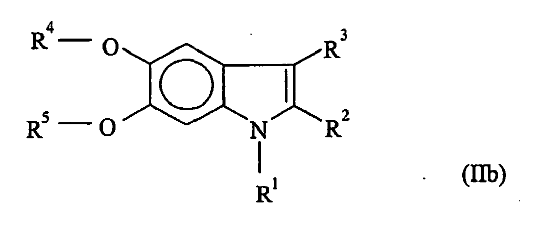 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Zusammensetzungen (A) oder (B) sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05 bis 10 Gew.-%, vorzugsweise 0,2 bis 5 Gew.-% enthalten.

In einer weiteren Ausführungsform kann es erfindungsgemäß bevorzugt sein, das Indolin- oder Indolderivat in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

In einer weiteren Ausführungsform enthalten die erfindungsgemäßen Zusammensetzungen (A) oder (B) mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1 -methylbenzol und 1 -Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methyl-resorcin, 5-Methylresorcin, 2,5-Dimethylresorcin und 2,6-Dihydroxy-3,4-dimethylpyridin.

Es ist bevorzugt, daß in der Ausführungsform als Färbemittel eine der Zusammensetzungen (A) und (B) kein Wasserstoffperoxid enthält und in dieser Wasserstoffperoxidfreien Zusammensetzung die direktziehenden Farbstoffe bzw. die Oxidationsfarbstoffvorprodukte enthalten sind.

Die erfindungsgemäßen Zusammensetzungen (A) und/oder (B) können weiterhin alle für solche Zubereitungen bekannten Wirk-, Zusatz- und Hilfsstoffe enthalten.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösemittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Liegt die Zusammensetzung (A) als Feststoff vor, so kann die Zusammensetzung (A) in Form eines Pulvers oder eines Formkörpers (d.h. eines Granulats, eines Extrudats oder eines Preßlings z.B. in Gestalt einer Tablette) konfektioniert werden.

Die Herstellung der erfindungsgemäßen Formkörper erfolgt zunächst durch das trockene Vermischen der Bestandteile, die ganz oder teilweise vorgranuliert sein können, und anschließendes Informbringen, insbesondere Verpressen zu Tabletten, wobei auf bekannte Verfahren zurückgegriffen werden kann. Zur Herstellung der erfindungsgemäßen Formkörper wird das Vorgemisch in einer sogenannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen.

Zunächst wird das Vorgemisch in die Matrize eingebracht, wobei die Füllmenge und damit das Gewicht und die Form des entstehenden Formkörpers durch die Stellung des unteren Stempels und die Form des Preßwerkzeugs bestimmt werden. Die gleichbleibende Dosierung auch bei hohen Formkörperdurchsätzen wird vorzugsweise über eine volumetrische Dosierung des Vorgemischs erreicht. Im weiteren Verlauf der Tablettierung berührt der Oberstempel das Vorgemisch und senkt sich weiter in Richtung des Unterstempels ab. Bei dieser Verdichtung werden die Partikel des Vorgemisches näher aneinander gedrückt, wobei das Hohlraumvolumen innerhalb der Füllung zwischen den Stempeln kontinuierlich abnimmt. Ab einer bestimmten Position des Oberstempels (und damit ab einem bestimmten Druck auf das Vorgemisch) beginnt die plastische Verformung, bei der die Partikel zusammenfließen und es zur Ausbildung des Formkörpers kommt. Je nach den physikalischen Eigenschaften des Vorgemisches wird auch ein Teil der Vorgemischpartikel zerdrückt, und es kommt bei noch höheren Drücken zu einer Sinterung des Vorgemischs. Bei steigender Preßgeschwindigkeit, also hohen Durchsatzmengen, wird die Phase der elastischen Verformung immer weiter verkürzt, so daß die entstehenden Formkörper mehr oder minder große Hohlräume aufweisen können. Im letzten Schritt der Tablettierung wird der fertige Formkörper durch den Unterstempel aus der Matrize herausgedrückt und durch nachfolgende Transporteinrichtungen wegbefördert. Zu diesem Zeitpunkt ist lediglich das Gewicht des Formkörpers endgültig festgelegt, da die Preßlinge aufgrund physikalischer Prozesse (Rückdehnung, kristallographische Effekte, Abkühlung etc.) ihre Form und Größe noch ändern können.

Die Tablettierung erfolgt in handelsüblichen Tablettenpressen, die prinzipiell mit Einfach- oder Zweifachstempeln ausgerüstet sein können. Im letzteren Fall wird nicht nur der Oberstempel zum Druckaufbau verwendet, auch der Unterstempel bewegt sich während des Preßvorgangs auf den Oberstempel zu, während der Oberstempel nach unten drückt. Für kleine Produktionsmengen werden vorzugsweise Exzentertablettenpressen verwendet, bei denen der oder die Stempel an einer Exzenterscheibe befestigt sind, die ihrerseits an einer Achse mit einer bestimmten Umlaufgeschwindigkeit montiert ist. Die Bewegung dieser Preßstempel ist mit der Arbeitsweise eines üblichen Viertaktmotors vergleichbar. Die Verpressung kann mit je einem Ober- und Unterstempel erfolgen, es können aber auch mehrere Stempel an einer Exzenterscheibe befestigt sein, wobei die Anzahl der Matrizenbohrungen entsprechend erweitert ist. Die Durchsätze von Exzenterpressen variieren ja nach Typ von einigen hundert bis maximal 3000 Tabletten pro Stunde.

Für größere Durchsätze wählt man Rundlauftablettenpressen, bei denen auf einem sogenannten Matrizentisch eine größere Anzahl von Matrizen kreisförmig angeordnet ist. Die Zahl der Matrizen variiert je nach Modell zwischen 6 und 55, wobei auch größere Matrizen im Handel erhältlich sind. Jeder Matrize auf dem Matrizentisch ist ein Ober- und Unterstempel zugeordnet, wobei wiederum der Preßdruck aktiv nur durch den Ober- bzw. Unterstempel, aber auch durch beide Stempel aufgebaut werden kann. Der Matrizentisch und die Stempel bewegen sich um eine gemeinsame senkrecht stehende Achse, wobei die Stempel mit Hilfe schienenartiger Kurvenbahnen während des Umlaufs in die Positionen für Befüllung, Verdichtung, plastische Verformung und Ausstoß gebracht werden. An den Stellen, an denen eine besonders gravierende Anhebung bzw. Absenkung der Stempel erforderlich ist (Befüllen, Verdichten, Ausstoßen), werden diese Kurvenbahnen durch zusätzliche Niederdruckstücke, Niederzugschienen und Aushebebahnen unterstützt. Die Befüllung der Matrize erfolgt über eine starr angeordnete Zufuhreinrichtung, den sogenannten Füllschuh, der mit einem Vorratsbehälter für das Vorgemisch verbunden ist. Der Preßdruck auf das Vorgemisch ist über die Preßwege für Ober- und Unterstempel individuell einstellbar, wobei der Druckaufbau durch das Vorbeirollen der Stempelschaftköpfe an verstellbaren Druckrollen geschieht.

Rundlaufpressen können zur Erhöhung des Durchsatzes auch mit zwei Füllschuhen versehen werden, wobei zur Herstellung einer Tablette nur noch ein Halbkreis durchlaufen werden muß. Zur Herstellung zwei- und mehrschichtiger Formkörper werden mehrere Füllschuhe hintereinander angeordnet, ohne daß die leicht angepreßte erste Schicht vor der weiteren Befüllung ausgestoßen wird. Durch geeignete Prozeßführung sind auf diese Weise auch Mantel- und Punkttabletten herstellbar, die einen zwiebelschalenartigen Aufbau haben, wobei im Falle der Punkttabletten die Oberseite des Kerns bzw. der Kernschichten nicht überdeckt wird und somit sichtbar bleibt. Auch Rundlauftablettenpressen sind mit Einfach- oder Mehrfachwerkzeugen ausrüstbar, so daß beispielsweise ein äußerer Kreis mit 50 und ein innerer Kreis mit 35 Bohrungen gleichzeitig zum Verpressen benutzt werden. Die Durchsätze moderner Rundlauftablettenpressen betragen über eine Million Formkörper pro Stunde.

Bei der Tablettierung mit Rundläuferpressen hat es sich als vorteilhaft erwiesen, die Tablettierung mit möglichst geringen Gewichtschwankungen der Tablette durchzuführen. Auf diese Weise lassen sich auch die Härteschwankungen der Tablette reduzieren. Geringe Gewichtschwankungen können auf folgende Weise erzielt werden:
- Verwendung von Kunststoffeinlagen mit geringen Dickentoleranzen
- Geringe Umdrehungszahl des Rotors
- Große Füllschuhe
- Abstimmung des Füllschuhflügeldrehzahl auf die Drehzahl des Rotors
- Füllschuh mit konstanter Pulverhöhe
- Entkopplung von Füllschuh und Pulvervorlage

Zur Verminderung von Stempelanbackungen bieten sich sämtliche aus der Technik bekannte Antihaftbeschichtungen an. Besonders vorteilhaft sind Kunststoffbeschichtungen, Kunststoffeinlagen oder Kunststoffstempel. Auch drehende Stempel haben sich als vorteilhaft erwiesen, wobei nach Möglichkeit Ober- und Unterstempel drehbar ausgeführt sein sollten. Bei drehenden Stempeln kann auf eine Kunststoffeinlage in der Regel verzichtet werden. Hier sollten die Stempeloberflächen elektropoliert sein.

Es zeigte sich weiterhin, daß lange Preßzeiten vorteilhaft sind. Diese können mit Druckschienen, mehreren Druckrollen oder geringen Rotordrehzahlen eingestellt werden. Da die Härteschwankungen der Tablette durch die Schwankungen der Preßkräfte verursacht werden, sollten Systeme angewendet werden, die die Preßkraft begrenzen. Hier können elastische Stempel, pneumatische Kompensatoren oder federnde Elemente im Kraftweg eingesetzt werden. Auch kann die Druckrolle federnd ausgeführt werden.

Im Rahmen der vorliegenden Erfindung geeignete Tablettiermaschinen sind beispielsweise erhältlich bei den Firmen Apparatebau Holzwarth GbR, Asperg, Wilhelm Fette GmbH, Schwarzenbek, Fann Instruments Company, Houston, Texas (USA), Hofer GmbH, Weil, Horn & Noack Pharmatechnik GmbH, Worms, IMA Verpackungssysteme GmbH Viersen, KILIAN, Köln, KOMAGE, Kell am See, KORSCH Pressen AG, Berlin, sowie Romaco GmbH, Worms. Weitere Anbieter sind beispielsweise Dr. Herbert Pete, Wien (AT), Mapag Maschinenbau AG, Bern (CH), BWI Manesty, Liverpool (GB), I. Holand Ltd., Nottingham (GB), Courtoy N.V., Halle (BE/LU) sowie Mediopharm Kamnik (SI). Besonders geeignet ist beispielsweise die Hydraulische Doppeldruckpresse HPF 630 der Firma LAEIS, D. Tablettierwerkzeuge sind beispielsweise von den Firmen Adams Tablettierwerkzeuge, Dresden, Wilhelm Fett GmbH, Schwarzenbek, Klaus Hammer, Solingen, Herber % Söhne GmbH, Hamburg, Hofer GmbH, Weil, Horn & Noack, Pharmatechnik GmbH, Worms, Ritter Pharamatechnik GmbH, Hamburg, Romaco, GmbH, Worms und Notter Werkzeugbau, Tamm erhältlich. Weitere Anbieter sind z.B. die Senss AG, Reinach (CH) und die Medicopharm, Kamnik (SI).

Das Verfahren zur Herstellung der Formkörper ist aber nicht darauf beschränkt, daß lediglich ein teilchenförmiges Vorgemisch zu einem Formkörper verpreßt wird. Vielmehr läßt sich das Verfahren auch dahingehend erweitern, daß man in an sich bekannter Weise mehrschichtige Formkörper herstellt, indem man zwei oder mehrere Vorgemische bereitet, die aufeinander verpreßt werden. Hierbei wird das zuerst eingefüllte Vorgemisch leicht vorverpreßt, um eine glatte und parallel zum Formkörperboden verlaufende Oberseite zu bekommen, und nach Einfüllen des zweiten Vorgemischs zum fertigen Formkörper endverpreßt. Bei drei- oder mehrschichtigen Formkörpern erfolgt nach jeder Vorgemisch-Zugabe eine weitere Vorverpressung, bevor nach Zugabe des letzten Vorgemischs der Formkörper endverpreßt wird.

Ein dritter Gegenstand der Erfindung ist eine wasserfreie Zusammensetzung, welche mindestens eine alkalisierend wirkende, feste Zusammensetzung des ersten Gegenstandes der Erfindung, sowie mindestens einen Bleich-Booster enthält.

Die erfindungsgemäße alkalisierend wirkende, feste Zusammensetzung ist in der wasserfreien Zusammensetzung bevorzugt in einer Menge von 1 bis 40 Gew.%, besonders bevorzugt in einer Menge von 2 bis 30 Gew.%, jeweils bezogen auf das Gewicht der gesamten wasserfreien Zusammensetzung, enthalten.

Es gilt für den Begriff Bleich-Booster und deren bevorzugten Vertreter das in diesem Zusammenhang im Rahmen des zweiten Gegenstandes, der Erfindung Gesagte. Die Bleich-Booster sind bevorzugt in einer Menge von 5 bis 60 Gew.-%, insbesondere in Mengen von 8 bis 30 Gew.-%, bezogen auf die gesamte wasserfreie Zusammensetzung enthalten.

Die wasserfreie Zusammensetzung kann optional Wasserstoffperoxid in Form von Anlagerungsprodukten von Wasserstoffperoxid an feste organische oder anorganische Verbindung enthalten. Die bevorzugten Vertreter dieser Anlagerungsprodukte wurden bereits im zweiten Gegenstand der Erfindung zusammengefasst.

Die erfindungsgemäße wasserfreie Zusammensetzung ist bevorzugt fest oder pastös. Es gilt für diese Ausführungsformen das im zweiten Gegenstand der Erfindung Gesagte.

Die erfindungsgemäße wasserfreie Zusammensetzung kann zusätzlich mindestens eine Verbindung ausgewählt aus Tensiden, konditionierenden Wirkstoffen, strukturverbessernden Wirkstoffen, und Wirk-, Hilfs- und Zusatzstoffen in bevorzugten Mengen enthalten. Es gilt für diese Ausführungsformen das im zweiten Gegenstand der Erfindung Gesagte.

Die erfindungsgemäße wasserfreie Zusammensetzung kann zusätzlich mindestens eine Verbindung aus der Gruppe der Entwicklerkomponenten, Kupplerkomponenten, direktziehenden Farbstoffe und Vorstufen naturanaloger Farbstoffe enthalten. Die bevorzugten Vertreter dieser Verbindungen wurden bereits im zweiten Gegenstand der Erfindung zusammengefasst.

Ein vierter Gegenstand der vorliegenden Erfindung ist ein Verfahren zur Stabilisierung von H₂O₂ während des Auflösevorgangs einer festen oder pastösen Zusammensetzung (A) in einer wäßrig oder wäßrig-alkoholischen Zusammensetzung (B), wobei die Zusammensetzung (A) als Alkalisierungmittel mindestens eine alkalisierend wirkende, feste Zusammensetzung des ersten Erfindungsgegenstandes enthält und mindestens eine der Zusammensetzungen (A) und (B) Wasserstoffperoxid enthält.

Es ist erfindungsgemäß bevorzugt, daß nur die Zusammensetzung (B) Wasserstoffperoxid enthält.

Für die Merkmale und die bevorzugten Ausführungsformen der Zusammensetzungen (A) und (B) gilt das für die wasserstoffperoxidhaltigen Mittel und deren Zusammensetzungen (A) und (B) des zweiten Gegenstandes der Erfindung Gesagte.

Für die Merkmale und die bevorzugten Ausführungsformen der alkalisierend wirkenden, festen Zusammensetzung gilt das für die Zusammensetzung des ersten Gegenstandes Gesagte.

Ein fünfter Gegenstand der vorliegenden Erfindung ist ein Verfahren zur Aufhellung keratinhaltiger Fasern, bei dem die Fasern mit einem der oben beschriebenen wasserstoffperoxidhaltigen Mittel des zweiten Gegenstandes der Erfindung behandelt werden.

Ein sechster Gegenstand der vorliegenden Erfindung ist ein Verfahren zur Färbung von keratinhaltigen Fasern, in dem die Fasern mit einem wasserstoffperoxidhaltigen Mittel des zweiten Gegenstandes der Erfindung in Gegenwart von direktziehenden Farbstoffen und/oder Oxidationsfarbstoffvorprodukten behandelt werden.

Ein siebter Gegenstand der vorliegenden Erfindung ist ein Verfahren zum Reinigen von Textilien und harten Oberflächen, bei dem die Textilien bzw. harten Oberflächen mit einem der oben beschriebenen wasserstoffperoxidhaltigen Mittel des zweiten Gegenstandes der Erfindung behandelt werden.

Die folgenden Beispiele sollen den Gegenstand der vorliegenden Erfindung erläutern ohne ihn jedoch zu beschränken.

### Beispiele

### 1.0 Herstellung eines erfindungsgemäßen Agglomerats 1

56.0 g Britesil^{®} C20¹ und 121 g Wasser werden gemischt. Unter Rühren werden zu dieser Mischung 0.4 g Turpinal^{®} SL² zugetropft. Die Mischung wird im Rotationsverdampfer (125 rpm) bei 80°C im Vakuum getrocknet. Die Mischung wird in ein Becherglas gegeben und für weitere 3 Tage im Trockenschrank bei 50°C getrocknet.
¹ Natriumsilikat (INCI-Bezeichnung: Sodium Silikate) (The PQ Corporation)
² 1-Hydroxyethan-1,1-diphosphonsäure (60 % Aktivsubstanz in Wasser) (INCl-Bezeichnung: Etidronic Acid, Aqua (Water)) (Solutia)

### 2.0 Verwendungsbeispiel in Blondierpulvern

| | |
|---|---|
| Blondierpulver 1 (erfindungsgemäß) | |
| Ammoniumperoxodisulfat | 13.00 g |
| Kaliumpersulfat | 42.80 g |
| Natriumpersulfat | 15.60 g |
| Agglomerat 1 (gemäß 1.0) | 28.20 g |
| Idranal^{®} III³ | 0.60 g |

| | |
|---|---|
| ³ Ethylendiamintetraessigsäure Dinatrium Salz·2 H₂O (INCl-Bezeichnung: Disodium EDTA) (Hersteller: Riedel De Haen) | |

### 3.0 Vergleichstests

Zum Vergleich mit dem erfindungsgemäßen Blondierpulver 1 gemäß 2.0 wurden folgende nicht erfindungsgemäße Blondierpulver hergestellt:

| Blondierpulver V1 | | Blondierpulver V2 | |
|---|---|---|---|
| Ammoniumperoxodisulfat | 13.00 g | Ammoniumperoxodisulfat | 13.00 g |
| Kaliumpersulfat | 42.80 g | Kaliumpersulfat | 42.80 g |
| Natriumpersulfat | 15.60 g | Natriumpersulfat | 15.60 g |
| Britesil^{®} C20 | 28.00 g | Britesil^{®} C20 | 28.00 g |
| Idranal^{®} III | 0.60 g | Idranal^{®} III | 0.60 g |
| | | Etidronsäure (fest) | 2.00 g |

Das Blondierpulver 1 enthält 28.02 Gew.-% Alkalisierungsmittel in Form von Britesil^{®} C20 im Agglomerat 1 und als Chelatbildner 0.12 Gew.-% Etidronsäure aus dem Agglomerat 1, sowie 0.60 Gew.-% EDTA Dinatriumsalz.

Das Blondierpulver V1 enthält 28.00 Gew.-% Alkalisierungmittel in Form von Britesil^{®} C20 und 0.60 Gew.% EDTA Dinatriumsalz als Chelatbildner.

Das Blondierpulver V2 enthält 27.45 Gew.-% Alkalisierungsmittel in Form von Britesil^{®} C20 und als Chelatbildner 0.59 Gew.-% EDTA Dinatriumsalz und 1.96 Gew.-% Etidronsäure.

### 3.1 Test zur Zersetzung des Wasserstoffperoxids beim Mischvorgang

In den folgenden Versuchen wurde als wasserstoffperoxidhaltige Creme die entsprechende wasserstoffperoxidhaltige Creme des Produkts Poly^{®} Intensiv Aufheller Ultra Plus (Schwarzkopf-Henkel) verwendet. Als Flasche dient das Vorratsbehältnis der wasserstoffperoxidhaltigen Creme des Marktprodukts.

### 3.1.1 Versuch 1

Zu jeweils 50.0 g einer wasserstoffperoxidhaltigen Creme werden 20.0 g einer der Blondierpulverrezepturen (1, V1 oder V2) in die Flasche gegeben und die Flasche verschlossen. Die Flasche wird 30 Sekunden lang geschüttelt. Dann wird nach einer Ruhezeit von 20 Sekunden die stehende Flasche geöffnet und die Zeit (Austrittszeit) gestoppt, bis der Flascheninhalt beginnt, durch die Gasentwicklung der Zersetzungsreaktion aus der Flasche auszutreten.

### 3.1.2 Versuch 2

Zu jeweils 50.0 g einer wasserstoffperoxidhaltigen Creme werden 20.0 g einer der Blondierpulverrezepturen (1, V1 oder V2) in die Flasche gegeben gegeben und die Flasche verschlossen. Die Flasche wird 30 Sekunden lang geschüttelt. Die Flasche bleibt weitere 30 Minuten verschlossen stehen. Nach dieser Ruhezeit wird die Flasche geöffnet und das Verhalten des Systems während der Öffnung beobachtet.

### 3.1.3 Versuchsbeobachtungen

### Blondierpulver 1 (erfindungsgemäß)

Versuch 1: Austrittszeit: 1 Stunde 55 Minuten
Versuch 2: Es zischt leicht beim Öffnen der Flasche. Es findet kein Produktaustritt während oder unmittelbar nach dem Öffnen der Flasche statt.

### Blondierpulver V1

Versuch 1: Austrittszeit: 29 Minuten
Versuch 2: Es zischt stark und spritzt beim Öffnen der Flasche. Es findet ein heftiger fontänenartiger Produktaustritt unmittelbar nach dem Öffnen der Flasche statt.

### Blondierpulver V2

Versuch 1: Austrittszeit: 38 Minuten
Versuch 2: Es zischt stark und spritzt beim Öffnen der Flasche. Es findet kein Produktaustritt während oder unmittelbar nach dem Öffnen der Flasche statt.

Obwohl das Blondierpulver V2 mehr Chelatbildner enthält (in Summe 2.55 Gew.-%) als das erfindungsgemäße Blondierpulver 1 (in Summe 0.72 Gew.-%), findet nach dem Mischvorgang des Blondierpulvers V2 mit der wasserstoffperoxidhaltigen Creme eine erhebliche Zersetzungsreaktion des Wasserstoffperoxids und eine daraus resultierende Gasentwicklung statt.

## Patentansprüche

1. Alkalisierend wirkende, feste Zusammensetzung, enthaltend
(i) in einer Menge von mindestens 75 Gew.% bezogen auf das Gewicht der Zusammensetzung ein Gemisch aus (a) mindestens einem partikulären Alkalisierungsmittel sowie (b) mindestens einem Chelatbildner,
(ii) gegebenenfalls weitere Zusatzstoffe,
wobei die Zusammensetzung aus Agglomeraten, gebildet aus (a), (b) und den optional enthaltenen Zusatzstoffen, besteht, **dadurch gekennzeichnet, daß** der Chelatbildner ausgewählt wird aus Ethylendiamintetraessigsäure, Diethylentriaminpentaessigsäure, 1-Hydroxyethan-1,1-diphosphonsäure (Etidronsäure), Dipicolinsäure, Acetanilid und Natriumstannat, sowie den physiologisch verträglichen Salzen der vorgenannten Säuren.

2. Zusammensetzung nach Anspruch 1, **dadurch gekennzeichnet, daß** das partikuläre Alkalisierungsmittel ausgewählt wird aus Ammonium-, Alkalimetall- und Erdalkalimetallhydroxiden, -carbonaten, -hydrogencarbonaten, -hydroxycarbonaten, -carbamiden, -silikaten, insbesondere -metasilikaten, sowie Alkaliphosphaten.

3. Zusammensetzung nach einem der Ansprüche 1 oder 2, **dadurch gekennzeichnet, daß** als partikuläres Alkalisierungmittel mindestens ein gegebenenfalls hydratisiertes Silikat der Formel (SiO₂)ₙ(Na₂O)ₘ(K₂O)ₚ enthalten ist, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

4. Zusammensetzung nach einem der Ansprüche 1 bis 3, **dadurch gekennzeichnet, daß** die partikulären Alkalisierungsmittel in einer Menge von 80 bis 99.8 Gew.-% bezogen auf die gesamte Zusammensetzung enthalten sind.

5. Zusammensetzung nach einem der Ansprüche 1 bis 4, **dadurch gekennzeichnet, daß** die Chelatbildner in einer Menge von 0.1 bis 20 Gew.-% bezogen auf die gesamte Zusammensetzung enthalten sind.

6. Zusammensetzung nach einem der Ansprüche 1 bis 5, **dadurch gekennzeichnet, daß** die Agglomerate einen mittleren Teilchendurchmesser von 10 bis 300 µm haben.

7. Zusammensetzung nach einem der Ansprüche 1 bis 6, **dadurch gekennzeichnet, daß** sie in Form eines Pulvers, Extrudats oder Granulats vorliegt.

8. Wasserstoffperoxidhaltiges Mittel, welches durch Mischen von mindestens zwei getrenntkonfektionierten Zusammensetzungen (A) und (B) erhalten wird, **dadurch gekennzeichnet, daß**
(i) die Zusammensetzung (A) wasserfrei ist und mindestens eine erfindungsgemäße, alkalisierend wirkende Zusammensetzung gemäß einem der Ansprüche 1 bis 7 enthält,
(ii) die Zusammensetzung (B) wäßrig oder wäßrig-alkoholisch ist und
(iii) mindestens eine der Zusammensetzungen (A) und (B) Wasserstoffperoxid enthält.

9. Mittel nach Anspruch 8, **dadurch gekennzeichnet, daß** die Zusammensetzung (A) zusätzlich mindestens einen Bleich-Booster enthält.

10. Mittel nach Anspruch 9, **dadurch gekennzeichnet, daß** der Bleich-Booster ausgewählt ist aus der Gruppe, die gebildet wird, aus (i) Peroxodisulfaten, Persulfaten und Perphosphaten des Ammoniums, der Alkali- und Erdalkalimetalle, (ii) Peroxiden der Alkali- und Erdalkalimetalle, (iii) Peroxocarborsäuren und deren physiologisch verträglichen Salzen und (iv) kationischen Nitrilen.

11. Mittel nach einem der Ansprüche 9 oder 10, **dadurch gekennzeichnet, daß** der Bleich-Booster ausgewählt wird aus Ammoniumperoxodisulfat, Kaliumperoxodisulfat, Natriumperoxodisulfat, Ammoniumpersulfat, Kaliumpersulfat, Natriumpersulfat, Kaliumperoxodiphosphat, Magnesiumperphthalat, Magnesiumperoxid und Bariumperoxid

12. Mittel nach einem der Ansprüche 8 bis 11, **dadurch gekennzeichnet, daß** die Zusammensetzung (B) eine Viskosität von 1 bis 100000 mPa·s besitzt (Brookfield RVT-Viskosimeter, Temperatur 20°C, Spindel Nr.4, 4 rpm).

13. Mittel nach einem der Ansprüche 8 bis 12, **dadurch gekennzeichnet, daß** die Zusammensetzung (B) eine Viskosität von 6000 bis 50000 mPa·s besitzt (Brookfield RVT-Viskosimeter, Temperatur 20°C, Spindel Nr.4, 4 rpm).

14. Mittel nach einem der Ansprüche 8 bis 13, **dadurch gekennzeichnet, daß** der pH-Wert der Zusammensetzung (B) in einem Bereich von pH 2 bis 7 liegt.

15. Mittel nach einem der Ansprüche 8 bis 14, **dadurch gekennzeichnet, daß** es einen pH-Wert von größer 7 besitzt.

16. Mittel nach einem der Ansprüche 8 bis 15, **dadurch gekennzeichnet, daß** es zusätzlich mindestens ein nichtionisches, anionisches, kationisches, zwitterionisches oder amphoteres Tensid enthält.

17. Mittel nach einem der Ansprüche 8 bis 16, **dadurch gekennzeichnet, daß** es zusätzlich mindestens einen konditionierenden Wirkstoff enthält.

18. Mittel nach einem der Ansprüche 8 bis 17, **dadurch gekennzeichnet, daß** es zusätzlich mindestens einen strukturverbessernden Wirkstoff enthält.

19. Mittel nach einem der Ansprüche 8 bis 18, **dadurch gekennzeichnet, daß** es zusätzlich mindestens einen direktziehenden Farbstoff und/oder mindestens ein Oxidationsfarbstoffvorprodukt vom Entwickler- und/oder Kupplertyp enthält.

20. Wasserfreie Zusammensetzung enthaltend eine alkalisierend wirkende, feste Zusammensetzung der Ansprüche 1 bis 7 und mindestens einen Bleich-Booster.

21. Verfahren zur Stabilisierung von H₂O₂ während des Auflösevorgangs einer festen oder pastösen Zusammensetzung (A) in einer wäßrigen oder wäßrig-alkoholischen Zusammensetzung (B), wobei die Zusammensetzung (A) als Alkalisierungsmittel mindestens eine Zusammensetzung gemäß einem der Ansprüche 1 bis 7 enthält und mindestens eine der Zusammensetzungen (A) und (B) Wasserstoffperoxid enthält.

22. Verfahren zur Aufhellung keratinhaltiger Fasern, bei dem die Fasern mit einem wasserstoffperoxidhaltingen Mittel nach einem der Ansprüche 8 bis 19 behandelt werden.

23. Verfahren zur Färbung von keratinhaltigen Fasern, in dem die Fasern mit einem wasserstoffperoxidhaltigen Mittel gemäß Anspruch 19 behandelt werden.

24. Verfahren zum Reinigen von Textilien und harten Oberflächen, bel dem die Textilien bzw. harten Oberflächen mit einem wasserstoffperoxidhaltigen Mittel nach einem der Ansprüche 8 bis 18 behandelt werden.

## Claims

1. A solid composition with an alkalising action, containing
(i) in an amount of at least 75% by weight based on the weight of the composition, a mixture of (a) at least one particulate alkalising agent as well as (b) at least one chelating agent,
(ii) optionally further additives,
wherein the composition consists of agglomerates formed from (a), (b) and the optionally contained additives, **characterized in that** the chelating agent is selected from ethylenediamine-tetraacetic acid, diethylenetriamine-pentaacetic acid, 1-hydroxyethane-1,1-diphosphonic acid (etidronic acid), dipicolinic acid, acetanilide, and sodium stannate, as well as physiologically acceptable salts of the aforementioned acids.

2. The composition according to claim 1, **characterized in that**, the particulate alkalising agent is selected from ammonium, alkaline metal and earth alkaline metal hydroxides, carbonates, hydrogencarbonates, hydroxycarbonates, carbamides, silicates, in particular metasilicates, as well as alkali phosphates.

3. The composition according to any of claims 1 or 2, **characterized in that** at least one optionally hydrated silicate of formula (SiO₂)ₙ(Na₂O)ₘ(K₂O)ₚ is contained as a particulate alkalising agent, wherein n represents a positive rational number and m and p represent independently of each other a positive rational number or 0, with the proviso that at least one of the parameters m or p is different from 0, and the ratio between n and the sum of m and p lies between 1:4 and 4:1.

4. The composition according to any of claims 1 to 4, **characterized in that** the particulate alkalising agents are contained in an amount from 80 to 99.8% by weight based on the total composition.

5. The composition according to any of claims 1 to 4, **characterized in that** the chelating agents are contained in an amount from 0.1 to 20% by weight based on the total composition.

6. The composition according to any of claims 1 to 5, **characterized in that** the agglomerates have an average particle diameter from 10 to 300 µm.

7. The composition according to any of claims 1 to 6, **characterized in that** it exists in the form of a powder, an extrudate or granulate.

8. A hydrogen peroxide containing agent which is obtained by mixing at least two separately made compositions (A) and (B), **characterized in that**
(i) the composition (A) is anhydrous and contains at least one composition according to the invention with an alkalising action according to any of claims 1 to 7,
(ii) the composition (B) is aqueous or aqueous-alcoholic and
(iii) at least one of the compositions (A) and (B) contains hydrogen peroxide.

9. The agent according to claim 8, **characterized in that** the composition (A) further contains at least one bleach booster.

10. The agent according to claim 9, **characterized in that** the bleach booster is selected from the group, which is formed from (i) peroxodisulfates, persulfates and perphosphates of ammonium, alkaline and earth alkaline metals, (ii) peroxide of alkaline and earth alkaline metals, (iii) peroxocarboxylic acids and their physiologically acceptable salts and (iv) cationic nitriles.

11. The agent according to any of claims 9 or 10, **characterized in that** the bleach booster is selected from ammonium peroxodisulfate, potassium peroxodisulfate, sodium peroxodisulfate, ammonium persulfate, potassium persulfate, sodium persulfate, potassium peroxodiphosphate, magnesium perphthalate, magnesium peroxide and barium peroxide.

12. The agent according to any of claims 8 to 11, **characterized in that** the composition (B) has a viscosity from 1 to 100,000 mPa.s (Brookfield RVT viscosimeter, temperature 20°C, spindle No. 4, 4 rpm).

13. The agent according to any of claims 8 to 12, **characterized in that** the composition (B) has a viscosity from 6,000 to 50,000 mPa.s (Brookfield RVT viscosimeter, temperature 20°C, spindle No. 4, 4 rpm).

14. The agent according to any of claims 8 to 13, **characterized in that** the pH value of the composition (B) lies in a pH range from 2 to 7.

15. The agent according to any of claims 8 to 14, **characterized in that** it has a pH value greater than 7.

16. The agent according to any of claims 8 to 15, **characterized in that** it further contains at least one non-ionic, anionic, cationic, zwitterionic or amphoteric surfactant.

17. The agent according to any of claims 8 to 16, **characterized in that** it further contains at least one conditioning active substance.

18. The agent according to any of claims 8 to 17, **characterized in that** it further contains at least one structure-enhancing active substance.

19. The agent according to any of claims 8 to 18, **characterized in that** it further contains at least one substantive dye and/or at least one oxidation dye precursor of the developer and/or coupler type.

20. An anhydrous composition containing a solid composition with an alkalising action according to claims 1 to 7, and at least one bleach booster.

21. A method for stabilising H₂O₂ during the dissolution process of a solid or pasty composition (A) in an aqueous or aqueous-alcoholic composition (B), wherein the composition A contains as an alkalising agent at least one composition according to any of claims 1 to 7 and at least one of the compositions (A) and (B) contains hydrogen peroxide.

22. A method for lightening keratinic fibers, wherein the fibers are treated with a hydrogen peroxide containing agent according to any of claims 8 to 19.

23. A method for dyeing keratinic fibers, wherein the fibers are treated with a hydrogen peroxide containing agent according to claim 19.

24. A method for cleaning textiles and hard surfaces, wherein the textiles or hard surfaces are treated with a hydrogen peroxide containing agent according to any of claims 8 to 18.

## Revendications

1. Composition solide à action alcalinisante contenant :
(i) en une quantité d'au moins 75 % en poids rapportés au poids de la composition, un mélange de (a) au moins un agent alcalinisant particulaire et de (b) au moins un agent chélatant ;
(ii) le cas échéant, d'autres additifs,
la composition étant constituée par des agglomérats formés à partir de (a), de (b) et des additifs contenus de manière facultative, **caractérisée en ce que** l'agent chélatant est choisi parmi l'acide éthylènediaminetétracétique, l'acide diéthylènetriaminepentacétique, l'acide 1-hydroxyéthane-1,1-diphosphonique (acide étidronique), l'acide dipicolinique, l'acétanilide et le stannate de sodium, ainsi que les sels physiologiquement acceptables des acides susmentionnés

2. Composition selon la revendication 1, **caractérisée en ce que** l'agent alcalinisant particulaire est choisi parmi des hydroxydes, des carbonates, des hydrogénocarbonates, des hydroxycarbonates, des carbamides, des silicates, en particulier des métasilicates d'ammonium, de métaux alcalins et de métaux alcalino-terreux, ainsi que des phosphates alcalins.

3. Composition selon l'une quelconque des revendications 1 ou 2, **caractérisée en ce qu'**elle contient, à titre d'agent alcalinisant particulaire, au moins un silicate éventuellement hydraté répondant à la formule (SiO₂)ₙ(Na₂O)ₘ(K₂O)ₚ dans laquelle n représente un nombre rationnel positif et m et p représentent, indépendamment l'un de l'autre, un nombre rationnel positif ou le nombre 0, avec les mesures qu'au moins un des paramètres m ou p est différent de 0 et que le rapport entre n et la somme de m et p se situe entre 1:4 et 4:1.

4. Composition selon l'une quelconque des revendications 1 à 3, **caractérisée en ce que** les agents alcalinisants particulaires sont contenus en une quantité de 80 à 99,8 % en poids rapportés à la composition totale.

5. Composition selon l'une quelconque des revendications 1 à 4, **caractérisée en ce que** les agents chélatants sont contenus en une quantité de 0,1 à 20 % en poids rapportés à la composition totale.

6. Composition selon l'une quelconque des revendications 1 à 5, **caractérisée en ce que** les agglomérats possèdent une granulométrie moyenne de 10 à 300 µm.

7. Composition selon l'une quelconque des revendications 1 à 6, **caractérisée en ce qu'**elle est présente sous la forme d'une poudre, d'un extrudat ou d'un produit de granulation.

8. Agent contenant du peroxyde d'hydrogène, que l'on obtient par mélange d'au moins deux compositions (A) et (B) confectionnées de manière séparée, **caractérisé en ce que**
(i) la composition (A) est anhydre et contient au moins une composition selon l'invention à action alcalinisante, selon l'une quelconque des revendications 1 à 7 ;
(ii) la composition (B) est aqueuse ou aqueuse-alcoolique ; et
(iii) au moins une des compositions (A) et (B) contient du peroxyde d'hydrogène.

9. Agent selon la revendication 8, **caractérisé en ce que** la composition (A) contient en outre au moins un activateur du blanchiment.

10. Agent selon la revendication 9, **caractérisé en ce que** l'activateur du blanchiment est choisi parmi le groupe qui est formé par (i) des peroxodisulfates, des persulfates et des perphosphates de l'ammonium, des métaux alcalins et des métaux alcalino-terreux, (ii) des peroxydes des métaux alcalins et des métaux alcalino-terreux, (iii) des acides peroxocarboxyliques et leurs sels physiologiquement acceptables et (iv) des nitriles cationiques.

11. Agent selon la revendication 9 ou 10, **caractérisé en ce que** l'activateur du blanchiment est choisi parmi le peroxodisulfate d'ammonium, le peroxodisulfate de potassium, le peroxodisulfate de sodium, le persulfate d'ammonium, le persulfate de potassium, le persulfate de sodium, le peroxodiphosphate de potassium, le perphtalate de magnésium, le peroxyde de magnésium et le peroxyde de baryum.

12. Agent selon l'une quelconque des revendications 8 à 11, **caractérisé en ce que** la composition (B) possède une viscosité de 1 à 100.000 mPa.s (viscosimètre Brookfield RVT, température 20 °C, broche n° 4, 4 tours/minute).

13. Agent selon l'une quelconque des revendications 8 à 12, **caractérisé en ce que** la composition (B) possède une viscosité de 6000 à 50.000 mPa.s (viscosimètre Brookfield RVT, température 20 °C, broche n° 4, 4 tours/minute).

14. Agent selon l'une quelconque des revendications 8 à 13, **caractérisé en ce que** la valeur de pH de la composition (B) se situe dans la plage de 2 à 7.

15. Agent selon l'une quelconque des revendications 8 à 14, **caractérisé en ce qu'**il possède une valeur de pH supérieure à 7.

16. Agent selon l'une quelconque des revendications 8 à 15, **caractérisé en ce qu'**il contient en outre au moins un agent tensioactif non ionique, anionique, cationique, zwitterionique ou amphotère.

17. Agent selon l'une quelconque des revendications 8 à 16, **caractérisé en ce qu'**il contient en outre au moins une substance active pour la revitalisation capillaire.

18. Agent selon l'une quelconque des revendications 8 à 17, **caractérisé en ce qu'**il contient en outre au moins une substance améliorant la texture.

19. Agent selon l'une quelconque des revendications 8 à 18, **caractérisé en ce qu'**il contient en outre au moins un colorant montant directement sur la fibre et/ou au moins un précurseur de colorant d'oxydation du type développeur et/ou du type coupleur.

20. Composition anhydre contenant une composition solide à action alcalinisante selon les revendications 1 à 7 et au moins un activateur du blanchiment.

21. Procédé pour la stabilisation du H₂O₂ au cours du processus de dissolution d'une composition solide ou pâteuse (A) dans une composition aqueuse ou aqueuse-alcoolique (B), la composition (A) contenant, à titre d'agent alcalinisant au moins une composition selon l'une quelconque des revendications 1 à 7 et au moins une des compositions (A) et (B) contenant du peroxyde d'hydrogène.

22. Procédé pour l'éclaircissement de fibres kératiniques, dans lequel on traite les fibres avec un agent contenant du peroxyde d'hydrogène selon l'une quelconque des revendications 8 à 19.

23. Procédé pour la coloration de fibres kératiniques, dans lequel on traite les fibres avec un agent contenant du peroxyde d'hydrogène, selon la revendication 19.

24. Procédé pour le nettoyage de textiles et de surfaces dures, dans lequel on traite les textiles, respectivement les surfaces dures avec un agent contenant du peroxyde d'hydrogène selon l'une quelconque des revendications 8 à 18.
